# EUROPÄISCHE PATENTANMELDUNG

(11) **EP 4 650 023 A1**
(43) Veröffentlichungstag der Anmeldung: **19.11.2025**
(21) Anmeldenummer: 25176707.5
(22) Anmeldetag: 15.05.2025
(51) Int. Cl.: B01B 1/00, A61L 2/20

(54) **VORRICHTUNG ZUM VERDAMPFEN EINER FLÜSSIGKEIT**

(30) Priorität: 16.05.2024 DE 102024113746
(71) Anmelder: GASTI Verpackungsmaschinen GmbH, 74523 Schwäbisch Hall (DE)
(72) Erfinder: BURGMEIER, Berthold, 89407 Dillingen (DE); KROGNER, Bastian, 74541 Vellberg (DE); STIEGLER, Achim, 74626 Bretzfeld (DE)
(74) Vertreter: Maiwald GmbH

(57) **Zusammenfassung**

Die vorliegende Erfindung betrifft eine Vorrichtung 10 zum Verdampfen einer Flüssigkeit, insbesondere einer Behandlungsflüssigkeit zur Sterilisation eines Behältnisses. Die Vorrichtung 10 umfasst ein Gehäuse 12, das einen Hohlraum 11 festlegt und eine Heizeinrichtung 14, mithilfe derer zumindest eine dem Hohlraum 11 zugewandte Innenoberfläche des Gehäuses 12 zumindest abschnittsweise beheizbar ist. Darüber hinaus umfasst die Vorrichtung 10 mindestens eine Einlassöffnung 16, 17 zum Zuführen von mit Druck beaufschlagter Luft in den Hohlraum 11 sowie mindestens eine Düse 30 zum Zuführen einer Behandlungsflüssigkeit in den Hohlraum 11, wobei bei bestimmungsgemäßer Verwendung der Vorrichtung 10 die Behandlungsflüssigkeit bei Kontakt mit der beheizten Innenoberfläche des Gehäuses 12 verdampft. Ebenso umfasst die Vorrichtung 10 eine Auslassöffnung 18 zum Abführen der verdampften Behandlungsflüssigkeit aus dem Hohlraum 11. Erfindungsgemäß erstreckt sich ein einen Durchgang festlegender Kanal 20 von der Auslassöffnung 18 in den Hohlraum 11, wobei der Kanal 20 sich bei bestimmungsgemäßer Verwendung der Vorrichtung 10 in vertikaler Richtung erstreckt.

## Beschreibung

### GEBIET DER ERFINDUNG

Die vorliegende Erfindung betrifft eine Vorrichtung zum Verdampfen einer Flüssigkeit, insbesondere einer Behandlungsflüssigkeit zur Sterilisation eines Behältnisses.

### HINTERGRUND DER ERFINDUNG

Lebensmittel, aber auch pharmazeutische Erzeugnisse, werden in unterschiedlichste Behältnisse abgefüllt. Die zu diesem Zweck eingesetzten Behältnisse können unterschiedlichste Formen wie auch Größen aufweisen. Zur Abfüllung von beispielsweise Lebensmitteln kommen relativ steife Becher oder Eimer, aber auch flexible Verpackungen, wie zum Beispiel Beutel, zum Einsatz. Essenziell dabei ist, dass die Behältnisse vor der Abfüllung sterilisiert werden, um die Sicherheit und Qualität der abgefüllten Produkte zu gewährleisten.

Bei der Sterilisation sollen insbesondere Mikroorganismen eliminiert werden, die ansonsten die Nahrungsmittel verderben oder Krankheiten verursachen könnten. Ebenso soll die Haltbarkeit verlängert werden, indem etwaige Keime abgetötet werden und eine mikrobielle Zersetzung verhindert wird.

Zur Sterilisation von Behältnissen in der Nahrungsmittelindustrie wird meist verdampftes Wasserstoffperoxid eingesetzt. Es wird davon ausgegangen, dass verdampftes Wasserstoffperoxid hochwirksam gegen alle bekannten Keime, einschließlich Bakteriensporen, ist und die Fähigkeit besitzt, Mikroorganismen abzutöten. Zur Verdampfung von Wasserstoffperoxid werden spezielle Verdampfer eingesetzt, die in regelmäßigen Abständen einem Reinigungsprozess mit Säure unterzogen werden.

Beim Verdampfen von Wasserstoffperoxid können Ablagerungen gebildet werden. Diese Ablagerungen entstehen oft durch Stabilisatoren, die dem Wasserstoffperoxid zugesetzt werden, um seine natürliche Zersetzung zu verhindern. Die Stabilisatoren können während des Verdampfungsprozesses Rückstände hinterlassen, die meist auf phosphathaltigen Verbindungen basieren. Die regelmäßige Reinigung mit Säure hilft, diese Ablagerungen zu entfernen und die Effizienz und Sicherheit des Verdampfers zu gewährleisten. Die Säure löst die Ablagerungen auf und verhindert, dass die Leistung des Verdampfers beeinträchtigt wird oder zu einer ungleichmäßigen Verteilung des Wasserstoffperoxids führt, was die Wirksamkeit des nachfolgenden Sterilisationsprozesses beeinträchtigen könnte.

Aus US 8,741,102 B2 ist ein Verdampfer für Wasserstoffperoxid bekannt, bei dem das eingespritzte flüssige Wasserstoffperoxid mithilfe von Druckluft an eine beheizte Wand eines zylindrischen Verdampfergehäuses bewegt wird. Da die Druckluft tangential in das Verdampfergehäuse eingebracht wird, wird das Gemisch aus Wasserstoffperoxid und Luft in eine schnelle Drehbewegung versetzt. Mit anderen Worten, es entsteht ein Zyklon innerhalb des Verdampfergehäuses. Das verdampfte Wasserstoffperoxid wird über eine Auslassöffnung abgeführt und einer Sterilisationskammer zugeführt. Zur Reinigung des Inneren des Verdampfergehäuses wird Säure über ein Rohr zugeführt, das sich von unten entlang der Zylinderachse in das Innere des Verdampfergehäuses erstreckt. Die Säure kann solange zugeführt werden, bis die Säureoberfläche die Höhe des axialen Rohres erreicht.

EP 2 455 106 A1 beschreibt ebenso einen Verdampfer zum Sterilisieren von Kunststoffbehältern. Dieser Verdampfer weist ein zylindrisches Gehäuse auf, dass diametral gegenüberliegende Öffnungen aufweist. An einer der Öffnungen ist ein um etwa 90° gekrümmter Rohrstutzen angebracht, der im Innern des zylindrischen Gehäuses verläuft und durch den mit Druck beaufschlagte Luft in das Innere des Gehäuses eingeblasen wird. Abschnittsweise innerhalb des Rohrstutzens verläuft eine Einspritzeinrichtung für Wasserstoffperoxid. Durch die Druckluft wird das Wasserstoffperoxid in Richtung einer im Innern des zylindrischen Gehäuses angeordneten beheizten Bodenplatte bewegt. Das verdampfte Wasserstoffperoxid wird über eine Auslassöffnung aus dem Gehäuse abgeführt. Ein in funktionaler Hinsicht dazu sehr ähnlicher Verdampfer ist in EP 2 407 181 A1 beschrieben.

In WO 2018/060422 A2 ist ein Verdampfer offenbart, der im Innern eine pyramidenförmig ausgebildete, Rillen aufweisende Verdampferoberfläche aufweist. In Richtung der Verdampferoberfläche wird das zu verdampfende Wasserstoffperoxid ausgegeben. Ebenso wird Luft in das Innere des Verdampfers eingeleitet, um über einen Auslass das verdampfte Wasserstoffperoxid abführen zu können.

### ZUSAMMENFASSUNG DER ERFINDUNG

Eine Aufgabe der vorliegenden Erfindung besteht darin, etwaige aus dem Stand der Technik bekannte Nachteile zu minimieren oder gar zu beseitigen. Insbesondere ist es eine Aufgabe der vorliegenden Erfindung, eine Vorrichtung zum Verdampfen einer Flüssigkeit, insbesondere einer Behandlungsflüssigkeit zur Sterilisation eines Behältnisses vorzuschlagen, mit Hilfe derer die Flüssigkeit nahezu gleichmäßig und vollständig verdampft und die auf einfache Weise vollständig, möglichst sogar automatisiert, gereinigt werden kann.

Diese Aufgabe und möglicherweise andere Aufgaben werden durch den Gegenstand des Patentanspruchs 1 gelöst. Optionale beziehungsweise bevorzugte Merkmale der Erfindung sind in den abhängigen Ansprüchen angegeben.

Die Erfindung betrifft eine Vorrichtung zum Verdampfen einer Flüssigkeit, insbesondere einer Behandlungsflüssigkeit zur Sterilisation eines Behältnisses. Die Vorrichtung umfasst ein Gehäuse, das einen Hohlraum festlegt und einen Heizanschluss, mithilfe dessen zumindest eine dem Hohlraum zugewandte Innenoberfläche des Gehäuses zumindest abschnittsweise beheizbar ist. Darüber hinaus umfasst die Vorrichtung mindestens eine Einlassöffnung zum Zuführen von mit Druck beaufschlagter Luft in den Hohlraum sowie mindestens eine Düse zum Zuführen einer Behandlungsflüssigkeit in den Hohlraum, wobei bei bestimmungsgemäßer Verwendung der Vorrichtung die Behandlungsflüssigkeit bei Kontakt mit der beheizten Innenoberfläche des Gehäuses verdampft. Ebenso umfasst die Vorrichtung eine Auslassöffnung zum Abführen der verdampften Behandlungsflüssigkeit aus dem Hohlraum. Erfindungsgemäß erstreckt sich ein einen Durchgang festlegender Kanal von der Auslassöffnung in den Hohlraum, wobei der Kanal sich bei bestimmungsgemäßer Verwendung der Vorrichtung in vertikaler Richtung erstreckt.

Das Gehäuse der erfindungsgemäßen Vorrichtung kann im Prinzip jedwede Form aufweisen. So kann die Querschnittsform des Gehäuses senkrecht zu seiner Längserstreckung kreisförmig, elliptisch, quadratisch, rechteckig oder auch polygonal sein. Darüber hinaus kann das Gehäuse zylindrisch, kegelstumpfförmig oder gar quaderförmig sein.

Die erfindungsgemäße Vorrichtung eignet sich zum Verdampfen jedweder Flüssigkeit, aber insbesondere solcher Flüssigkeiten, die bei der Sterilisation von Behältnissen verwendet werden, welche beispielsweise in der Nahrungsmittelindustrie oder auch in der pharmazeutischen Industrie eingesetzt werden. Mit Hilfe der Vorrichtung wird vorteilhafterweise Wasserstoffperoxid verdampft.

Die Heizeinrichtung, mithilfe derer zumindest die Innenoberfläche des Gehäuses zumindest abschnittsweise, aber auch vollständig, beheizt werden kann, kann grundsätzlich beliebiger Art sein. So ist eine elektrische Heizung denkbar, bei der die Innenoberfläche des Gehäuses durch eine Heizspindel auf eine vorbestimmte Temperatur aufgeheizt werden kann. Es ist aber auch der Einsatz von Dampf denkbar, der in Kontakt mit dem Gehäuse gebracht wird, sodass sich dessen Innenoberfläche auf eine für die spezielle Anwendung geeignete Temperatur erwärmen kann.

Die mit Druck beaufschlagte Luft, die durch die Einlassöffnung in den Hohlraum ausgegeben wird, kann einen Druck in einem Bereich von 0,05 bis 6 bar besitzen. Die Luft ist dabei vorteilhafterweise sterile Luft.

Durch den Kanal, der sich von der Auslassöffnung in das Innere des Gehäuses erstreckt, wird das Gehäuseinnere in zwei fluiddynamisch getrennte Bereiche unterteilt, nämlich einerseits in einen Bereich außerhalb des Kanals und andererseits einen Bereich innerhalb des Kanals. Der Bereich außerhalb des Kanals bildet sozusagen einen Zwischenraum zwischen der beheizbaren Innenwand des Gehäuses und der Außenseite des Kanals. Die durch die Düse in den Hohlraum und damit in den Zwischenraum des Gehäuses zugeführte Behandlungsflüssigkeit wird mithilfe von Druck oder mit Druck beaufschlagter Luft zerstäubt und in dem Zwischenraum in Bewegung versetzt, die umso schneller ist, je kleiner der Abstand zwischen der Innenoberfläche des Gehäuses und der Außenseite des Kanals ist. Durch diese schnelle Bewegung der mit Druck beaufschlagten Luft wird die zu verdampfende Behandlungsflüssigkeit auf sehr effektive Weise an die beheizte Innenwand bewegt und dort gleichmäßig verteil, wodurch die Tropfen der zerstäubten Behandlungsflüssigkeit mit einem möglichst großen Teil der beheizten Innenoberfläche des Gehäuses in Kontakt treten können, und wodurch die Behandlungsflüssigkeit sehr gleichmäßig im Hohlraum des Gehäuses verdampfen kann. Durch den Kanal wird sodann die verdampfte Behandlungsflüssigkeit aus der Vorrichtung abgeführt. Die vertikale Erstreckung des Kanals erlaubt eine vertikale Aufstellung der Vorrichtung und ist in vielerlei Hinsicht vorteilhaft.

Gemäß einer Ausführungsform der Erfindung besitzt der Kanal in einem Querschnitt senkrecht zur Längserstreckung des Kanals eine Umfangslänge, die entlang des Kanals variiert. Durch die Änderung des Kanalquerschnittes entlang der Längserstreckung des Kanals ändert sich entlang des Kanals der Abstand zwischen der Außenwand des Kanals und der Innenoberfläche des Gehäuses. Hierdurch verändert sich die Bewegungsgeschwindigkeit des Gemisches aus mit Druck beaufschlagter Luft (Trägerluft) und zerstäubter und teilweise auch bereits verdampfter Behandlungsflüssigkeit, wodurch etwaige Wandreibungseffekte einerseits im Bereich des Gehäuses und andererseits im Bereich der Außenwand des Kanals berücksichtigt, mitunter sogar ausgeglichen werden können.

Gemäß einer Ausführungsform der Erfindung nimmt die Umfangslänge entlang des Kanals mit zunehmendem Abstand von der Auslassöffnung zu. Dies ist insbesondere dann von Vorteil, wenn beispielsweise das Gehäuse zylindrisch ist und hierdurch der Abstand zwischen der Außenwand des Kanals und der Innenoberfläche des Gehäuses entlang des Kanals abnimmt. Aufgrund des Bernoulli-Effekts kommt es dann zu einer Erhöhung der Bewegungsgeschwindigkeit des Gemisches aus mit Druck beaufschlagter Luft (Trägerluft) und Behandlungsflüssigkeit. Simulationsmessungen haben allerdings ergeben, dass aufgrund dieser Geometrieänderung des Kanals die Bewegungsgeschwindigkeit des Gemisches aus mit Druck beaufschlagter Luft (Trägerluft) und Behandlungsflüssigkeit entlang des Kanals mehr oder weniger konstant gehalten werden kann, da etwaige Wandreibungseffekte versuchen, die Bewegungsgeschwindigkeit des Gemisches aus mit Druck beaufschlagter Luft (Trägerluft) und Behandlungsflüssigkeit zu verringern.

Gemäß einer Ausführungsform der Erfindung ist der Kanal abschnittsweise zylinderförmig und/oder abschnittsweise kegelstumpfförmig. Auch hierdurch kann gezielt auf die Bewegungsgeschwindigkeit des Gemisches aus mit Druck beaufschlagter Luft (Trägerluft) und Behandlungsflüssigkeit mit zunehmender Verweildauer innerhalb der Vorrichtung und entlang des Kanals Einfluss genommen werden.

Gemäß einer Ausführungsform der Erfindung erstreckt sich der Kanal über eine Längserstreckung L_{Kanal}, die in einem Bereich von 0 < L_{Kanal} < 0,9L_{Hohlraum} liegt, wobei der Wert L_{Kanal} die Länge des Kanals gemessen von der Auslassöffnung und L_{Hohlraum} die Länge der Längserstreckung des Hohlraumes der Vorrichtung ist. Vorteilhaft ist es, wenn sich der Kanal von der Auslassöffnung bis nahezu an das gegenüberliegende Hohlraumende der Vorrichtung erstreckt, weil hierdurch das Gemisch aus mit Druck beaufschlagter Luft (Trägerluft) und Behandlungsflüssigkeit die längste Verweildauer im Bereich der beheizten Innenoberfläche des Gehäuses hat und nicht schon beispielsweise vorher durch den Kanal hindurch zur Auslassöffnung gelangen kann. Im Prinzip sind aber auch kürzere Kanallängen denkbar, wenngleich diese mitunter aus fluiddynamischer Hinsicht und im Sinne einer gleichförmigen und vollständigen Verdampfung eher weniger vorteilhaft sein dürften. Folgende Kanallängen sind ebenso denkbar: 0 < L_{Kanal} < 0,8L_{Hohlraum}, 0 < L_{Kanal} < 0,7L_{Hohlraum}, 0 < L_{Kanal} < 0,6L_{Hohlraum}, 0 < L_{Kanal} < 0,5L_{Hohlraum}.oder kleiner.

Gemäß einer Ausführungsform der Erfindung umfasst die Vorrichtung des Weiteren eine Reinigungsmitteleinlassöffnung zum Zuführen von Reinigungsmittel in den Hohlraum. Da sich während der Verdampfung der Behandlungsflüssigkeit, insbesondere wenn Wasserstoffperoxid innerhalb der Vorrichtung verdampft wird, Ablagerungen bilden können, muss die Vorrichtung in regelmäßigen Abständen von innen gereinigt werden. Zu diesem Zweck umfasst die Vorrichtung eine Einlassöffnung für Reinigungsmittel, durch die Reinigungsmittel auf einfache Weise in den Hohlraum eingebracht werden kann.

Gemäß einer Ausführungsform der Erfindung ist der Durchgang des Kanals der Reinigungsmitteleinlassöffnung zugewandt. Eine derartige Ausführungsform ist insbesondere dahingehend vorteilhaft, da Reinigungsmittel, ggf. unter Druck, in den Hohlraum zugeführt werden kann, wodurch sich sowohl der Zwischenraum als auch der Raum innerhalb des Kanals mit Reinigungsmittel füllt. Das Reinigungsmittel kann dann durch die Auslassöffnung abgesaugt werden, wodurch auch der gesamte Rohrtrakt, der sich bei bestimmungsgemäßer Verwendung der Vorrichtung an die Auslassöffnung anschließt und zur Sterilisationskammer führt, gleichzeitig mit gereinigt werden kann.

Gemäß einer Ausführungsform der Erfindung ist die mindestens eine Einlassöffnung mit einem Einlassrohr verbunden, dass mit einer Einrichtung zu Bereitstellung von mit Druck beaufschlagter Luft fluidmäßig verbunden ist, wobei die mindestens eine Düse koaxial und innerhalb des Einlassrohres angeordnet ist. Hierdurch wird auf sehr effiziente Weise erreicht, dass unmittelbar nach dem Austreten aus der Düse die Behandlungsflüssigkeit durch die Druckluft verwirbelt und zerstäubt wird. Hierdurch bildet sich sozusagen unmittelbar nach der Düse sprayförmige Behandlungsflüssigkeit, die sich sodann mit hoher Geschwindigkeit innerhalb des Hohlraumes der Vorrichtung weiterbewegt.

Gemäß einer Ausführungsform der Erfindung weist das Einlassrohr oder die mindestens eine Einlassöffnung im Bereich der mindestens einen Düse eine Querschnittsverengung auf. Durch die Querschnittsverengung wird die aus der Düse austretende Behandlungsflüssigkeit noch zusätzlich beschleunigt, mitunter aufgrund des dabei auftretenden Venturi-Effekts auf sehr hohe Geschwindigkeiten.

Gemäß einer Ausführungsform ist die mindestens eine Einlassöffnung so angeordnet, dass die mit Druck beaufschlagte Luft, welche die mindestens eine Einlassöffnung verlässt, tangential zur Innenoberfläche des Gehäuses in den Hohlraum eintritt. Durch das Ausgeben der mit Druck beaufschlagten Luft in tangentialer Richtung relativ zur gekrümmten Gehäusewand, wird unmittelbar nach Austreten in den Hohlraum der Vorrichtung die Luft bereits in eine Drehbewegung versetzt, wodurch die Behandlungsflüssigkeit, welche durch die Luft quasi mitgerissen wird, ebenfalls in eine Drehbewegung versetzt wird und in Kontakt mit einer großen Fläche der beheizten Innenoberfläche des Gehäuses der Vorrichtung gebracht wird.

Gemäß einer Ausführungsform sind mindestens zwei gegenüberliegende Einlassöffnungen vorgesehen, wobei jede Einlassöffnung der mindestens zwei Einlassöffnungen so angeordnet ist, dass die mit Druck beaufschlagte Luft, welche die Einlassöffnung verlässt, tangential zur Innenoberfläche des Gehäuses in den Hohlraum eintritt. Dadurch, dass zwei gegenüberliegende Einlassöffnungen vorgesehen sind, durch die jeweils mit Druck beaufschlagte Luft in den Hohlraum der Vorrichtung ausgegeben wird, kann die Drehbewegung des Gemisches aus Behandlungsflüssigkeit und mit Druck beaufschlagter Luft weiter verstärkt werden.

Gemäß einer Ausführungsform der Erfindung ist die mindestens eine Düse in einem Abstand von der Innenoberfläche des Gehäuses innerhalb des Hohlraumes so angeordnet, dass ein die mindestens eine Düse verlassender hypothetischer Düsenstrahl senkrecht auf die Innenoberfläche des Gehäuses trifft. Durch das senkrechte Auftreffen der Behandlungsflüssigkeit auf die beheizte Innenoberfläche des Gehäuses wird die Flugzeit der Behandlungsflüssigkeit von der Ausgabe aus der Düse bis zum Auftreffen auf der Innenoberfläche des Gehäuses auf ein Minimum verkürzt, sodass die Behandlungsflüssigkeit sehr schnell in Kontakt mit der beheizten Innenoberfläche des Gehäuses tritt und rasch verdampft werden kann.

Gemäß einer Ausführungsform der Erfindung ist eine Vielzahl von Düsen an einer Düsenlanze angeordnet, wobei die Düsenlanze sich bei bestimmungsgemäßer Verwendung der Vorrichtung in vertikaler Richtung erstreckt und innerhalb des Hohlraumes angeordnet ist, und wobei die Vielzahl von Düsen in einem Abstand von der Innenoberfläche des Gehäuses angeordnet ist. Bei dieser Ausführungsform wird die Behandlungsflüssigkeit nicht über eine an der Gehäusewand vorgesehene Düse in den Hohlraum ausgegeben, sondern über eine Düsenlanze, die mehrere Düsen aufweist. Die Düsenlanze erstreckt sich dabei in vertikaler Richtung und in einem Abstand zur Innenoberfläche des Gehäuses. Da durch die Düsenlanze Behandlungsflüssigkeit an mehreren Stellen in vertikaler Richtung verteilt ausgegeben werden kann, kann so die Konzentration der verdampften Behandlungsflüssigkeit über die Höhe (Länge) des Hohlraumes homogener gestaltet werden.

Gemäß einer Ausführungsform der Erfindung ist die Vielzahl von Düsen an der Düsenlanze so angeordnet, dass eine Vielzahl von hypothetischen Düsenstrahlen, die aus der Vielzahl von Düsen austreten, senkrecht auf die Innenoberfläche des Gehäuses treffen. Hierdurch wird die Flugzeit der Behandlungsflüssigkeit, die aus jeder der Düsen ausgegeben wird, auf ein Minimum verkürzt.

Gemäß einer Ausführungsform der Erfindung sind sämtliche Düsen der Düsenlanze mit lediglich einer innerhalb der Düsenlanze verlaufenden Versorgungsleitung fluidmäßig verbunden. Hierdurch wird der Aufbau der Düsenlanze stark vereinfacht, da jede Düse über eine gemeinsame Versorgungsleitung mit Behandlungsflüssigkeit versorgt werden kann.

Gemäß einer Ausführungsform der Erfindung weist die Düsenlanze eine Kühlleitung auf zum Kühlen der Düsenlanze. Aufgrund der vorherrschend hohen Temperaturen im Hohlraum der Vorrichtung, in die sich die Düsenlanze erstreckt, ergeben sich auch zunehmend Gefahren, die von der Behandlungsflüssigkeit im Hohlraum ausgehen können. Wird beispielsweise Wasserstoffperoxid als Behandlungsflüssigkeit verwendet, ist die Explosionsgefahr stark erhöht, da sich innerhalb der Düsenlanze das Wasserstoffperoxid noch in flüssiger Form befindet.

Gemäß einer Ausführungsform der Erfindung ist das Gehäuse doppelwandig mit einem Zwischenraum ausgebildet, wobei der Heizanschluss mit dem Zwischenraum fluidmäßig verbunden ist zum Einführen von Wasserdampf in den Zwischenraum und zum zumindest abschnittsweisen Aufheizen von zumindest der Innenoberfläche des Gehäuses. Durch die doppelwandige Ausführung des Gehäuses kann Dampf in den Zwischenraum eingeführt werden. Eine Aufheizung mittels Dampf ist von Vorteil, da es zu einer gleichmäßigeren Aufheizung des Gehäuses und somit zu einer homogeneren Temperaturverteilung führt, was wiederum im Sinne einer gleichmäßigen und homogenen Verdampfung der Behandlungsflüssigkeit von Vorteil ist.

Gemäß einer Ausführungsform der Erfindung umfasst die Vorrichtung des Weiteren einen Temperaturfühler, dessen Fühlerspitze sich in den Zwischenraum des doppelwandigen Gehäuses erstreckt. Die Temperaturfühlerspitze kann die Temperatur des Dampfes zu jeder Zeit erfassen, die sodann wiederum als Regelgröße einem außerhalb der Vorrichtung befindlichen Regelkreis zugeführt werden kann.

Gemäß einer Ausführungsform der Erfindung ist das Gehäuse zylindrisch ausgebildet. Ein zylindrisches Gehäuse ist in praktischer Hinsicht vorteilhaft und verglichen mit anderen Gehäuseformen relativ einfach und kostengünstig herzustellen.

Gemäß einer Ausführungsform der Erfindung ist die mindestens eine Düse zum Zuführen von Wasserstoffperoxid in den Hohlraum ausgebildet. Die Verwendung von Wasserstoffperoxid als Behandlungsflüssigkeit ist dahingehend von Vorteil, als dass Wasserstoffperoxid zur Sterilisation von Behältnissen in der Lebensmittel- und der pharmazeutischen Industrie weitläufig eingesetzt wird und die Vorrichtung daher eine breite Anwendung finden kann.

Die vorstehend beschriebenen vorteilhaften Merkmale der Erfindung können in beliebiger Weise miteinander kombiniert werden, unabhängig davon, welchen Aspekt der Erfindung sie betreffen.

Diese und andere Ausführungsformen der vorliegenden Erfindung werden nun rein beispielhaft anhand der beigefügten Zeichnungen beschrieben.

### KURZE BESCHREIBUNG DER ZEICHNUNGEN

Die Erfindung wird nun rein beispielhaft unter Bezugnahme auf die Zeichnungen und anhand bevorzugter Ausführungsformen der Erfindung näher erläutert. Von den Figuren zeigen:
- Fig. 1: eine schematische perspektivische Ansicht einer Vorrichtung zum Verdampfen einer Flüssigkeit, insbesondere einer Behandlungsflüssigkeit zur Sterilisation eines Behältnisses, gemäß einer bevorzugten Ausführungsform der Erfindung;
- Fig. 2: eine teilweise in vertikaler Richtung geschnittene Ansicht der in Fig. 1 dargestellten Vorrichtung;
- Fig. 3: eine in vertikaler Richtung geschnittene Ansicht der in Fig. 1 dargestellten Vorrichtung im Bereich der Einlassöffnung für die Zuführung von mit Druck beaufschlagter Luft in das Innere der Vorrichtung;
- Fig. 4: eine teilweise in vertikaler Richtung geschnittene Ansicht einer Vorrichtung gemäß einer noch weiteren bevorzugten Ausführungsform der Erfindung, die eine alternative Düse zur Einbringung der zu verdampfenden Flüssigkeit in das Innere der Vorrichtung umfasst;
- Fig. 5: eine schematische perspektivische Ansicht einer Vorrichtung zum Verdampfen einer Flüssigkeit, insbesondere einer Behandlungsflüssigkeit zur Sterilisation eines Behältnisses, gemäß einer weiteren bevorzugten Ausführungsform der Erfindung; und
- Fig. 6: eine in vertikaler Richtung geschnittene Ansicht der in Fig. 5 dargestellten Vorrichtung.

### DETAILLIERTE BESCHREIBUNG BEVORZUGTER AUSFÜHRUNGSFORMEN

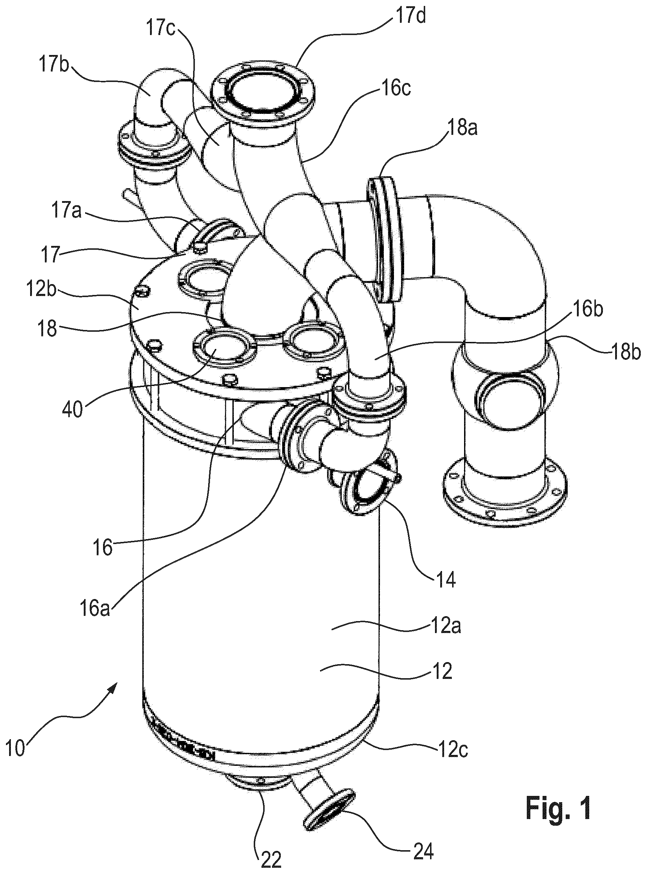

In den Figuren 1 bis 6 weisen identische oder einander entsprechende Bezugszeichen, die beispielsweise durch ein Asterisk ergänzt sein können, auf identische oder in funktionaler Hinsicht auf einander entsprechende Komponenten bzw. Bauteile der Vorrichtung hin.

Die erfindungsgemäße Vorrichtung eignet sich zum Verdampfen jedweder Flüssigkeiten, insbesondere solcher Flüssigkeiten, die bei der Sterilisation von Behältnissen verwendet werden, welche beispielsweise in der Nahrungsmittelindustrie oder auch in der pharmazeutischen Industrie eingesetzt werden. Mit Hilfe der Vorrichtung wird vorteilhafterweise Wasserstoffperoxid verdampft, das im Anschluss einer Sterilisationskammer zugeführt wird, in der die Behältnisse sodann sterilisiert werden. Solche Behältnisse umfassen zum Beispiel eigensteife Becher, Eimer, Vials, Dosen oder Flaschen, aber auch flexible Beutel.

Die erfindungsgemäße Vorrichtung kann beim bestimmungsgemäßen Gebrauch sowohl in einer aufrechten (vertikalen) Position, einer waagerechten (horizontalen) Position, einer umgekehrt aufrechten (umgekehrt vertikalen), einer sog. auf den Kopf gestellten Position, oder einer in einem beliebigen Winkel zur Horizontalen geneigten Position auf einem ebenen Untergrund installiert werden.

Das Gehäuse der erfindungsgemäßen Vorrichtung kann im Prinzip jedwede Form aufweisen. So kann die Querschnittsform des Gehäuses senkrecht zu seiner Längserstreckung kreisförmig, elliptisch, quadratisch, rechteckig oder auch polygonal sein. Darüber hinaus kann das Gehäuse zylindrisch, kegelstumpfförmig oder gar quaderförmig sein.

Fig. 1 zeigt eine perspektivische Ansicht einer Vorrichtung 10 gemäß einer bevorzugten Ausführungsform der Erfindung zum Verdampfen einer Flüssigkeit, insbesondere einer Behandlungsflüssigkeit zur Sterilisation eines Behältnisses, und insbesondere einer Behandlungsflüssigkeit in Form von Wasserstoffperoxid.

Die Vorrichtung 10 umfasst ein Gehäuse 12, das auf vorteilhafte Weise zylindrisch ausgebildet ist. Das zylindrische Gehäuse 12 umfasst eine Wandung, die einen zylindrischen Mantel (Gehäusewand) 12a und zwei gegenüberliegende Stirnseiten 12b, 12c umfasst. Eine der beiden Stirnseiten 12b, 12c oder beide Stirnseiten 12b, 12c können planar oder auch leicht gewölbt ausgebildet sein.

Das Gehäuse 12 umfasst bei der in Fig. 1 gezeigten Ausführungsform zwei Öffnungen 16, 17, welche die Wandung des Gehäuses 12 durchdringen und an dafür geeigneten Stellen, auf vorteilhafte Weise an diametral gegenüberliegenden Stellen an der Gehäusewand 12a vorgesehen sind. Durch die beiden Öffnungen 16, 17 wird mit Druck beaufschlagte Luft, insbesondere sterile Luft, in das Innere 11 des Gehäuses 12 eingebracht. Der Druck der in das Innere 11 des Gehäuses 12 eingebrachten Luft hängt von verschiedenen Faktoren ab, wie zum Beispiel der Größe des Gehäuses 12, der Umgebungstemperatur oder auch von der Art der zu verdampfenden Behandlungsflüssigkeit. Wenn in dieser Anmeldung von Druckluft die Rede ist, so kann die Luft auch von einem Ventilator, und nicht notwendigerweise von einem Kompressor mit Druck beaufschlagt werden.

Im Sinne der Erfindung ist auch denkbar, lediglich eine Öffnung 16, aber auch mehr als zwei Öffnungen 16, 17 für die Zufuhr von mit Druck beaufschlagter Luft in das Innere 11 des Gehäuses 12 vorzusehen.

In die Öffnungen 16, 17 sind entsprechend dimensioniert Rohrabschnitte mit oder ohne einem endseitigen Flansch 16a, 17a so eingesetzt, dass sie die Öffnungen 16, 17 dichtend umgeben. Weitere Rohrabschnitte 16b, 17b sind mit den Flanschen 16a, 17a verbunden. Die Geometrie der weiteren Rohrabschnitte 16b, 17b kann variieren und ist an den jeweiligen Einsatzort der Vorrichtung 10 und den örtlichen Begebenheiten angepasst. Von den Rohrabschnitten 16b, 17b führen weitere Rohrabschnitte 16c, 17c zu einem gemeinsamen Flansch 17d, der mit einer Druckluftquelle (nicht gezeigt) verbunden ist, welche Luft mit für den Einsatzzweck geeignetem Druck zur Verfügung stellt.

Darüber hinaus ist mittig an der oberen Stirnseite 12b des zylindrischen Gehäuses 12 eine Öffnung 18 vorgesehen, in die ebenfalls ein Rohrabschnitt mit endseitigem Flansch 18a dichtend eingesetzt ist, und über die das verdampfte Wasserstoffperoxid aus dem Inneren 11 des Gehäuses 12 herausgeführt werden kann. Je nach Einsatzort der Vorrichtung 10 und den örtlichen Begebenheiten kann ein weitere Rohrabschnitt 18b an den Rohrabschnitt 18a angeflanscht sein.

Gemäß Fig. 1 sind an der oberen Stirnseite 12b des zylindrischen Gehäuses 12 ebenso Schaugläser 40 angeordnet, durch die das Innere 11 des Gehäuses 12 von außen sichtbar ist.

An der unteren Stirnseite 12c, vorteilhafterweise mittig an der unteren Stirnseite 12c, des zylindrischen Gehäuses 12 ist ein Anschluss 22 zur Zuführung von Reinigungsflüssigkeit in das Innere 11 des Gehäuses 12 vorgesehen. Da sich beim Verdampfen von Wasserstoffperoxid aufgrund der darin enthaltenen Stabilisatoren Ablagerungen im Innern 11 des Gehäuses 12 bilden können, muss das Innere 11 des Gehäuses 12 in regelmäßigen Abständen mit Hilfe einer säurehaltigen Reinigungsflüssigkeit gereinigt werden.

An der Gehäusewand 12a des Gehäuses 12 ist ein Heizanschluss 14 vorgesehen, mit Hilfe dessen zumindest die Innenoberfläche des Gehäuses 12 auf eine gewünschte Temperatur erwärmt bzw. aufgeheizt werden kann. Zur Erwärmung der Innenoberfläche des Gehäuses 12 wird auf vorteilhafte Weise Wasserdampf verwendet.

Da die Gehäusewand 12a und die untere Stirnseite 12c doppelwandig mit einem dazwischenliegenden Zwischenraum 12d ausgebildet sind, wird der Wasserdampf zur Erwärmung der Innenoberfläche des Gehäuses 12 in den Zwischenraum 12d eingebracht. Kondensierter Wasserdampf kann über einen Auslass 24 aus dem Zwischenraum 12d herausgeführt werden. Der Auslass 24 sollte an einer dafür geeigneten Position vorgesehen werden, die vorteilhafterweise sich an einem möglichst tiefgelegenen Punkt des Zwischenraumes 12d befinden sollte.

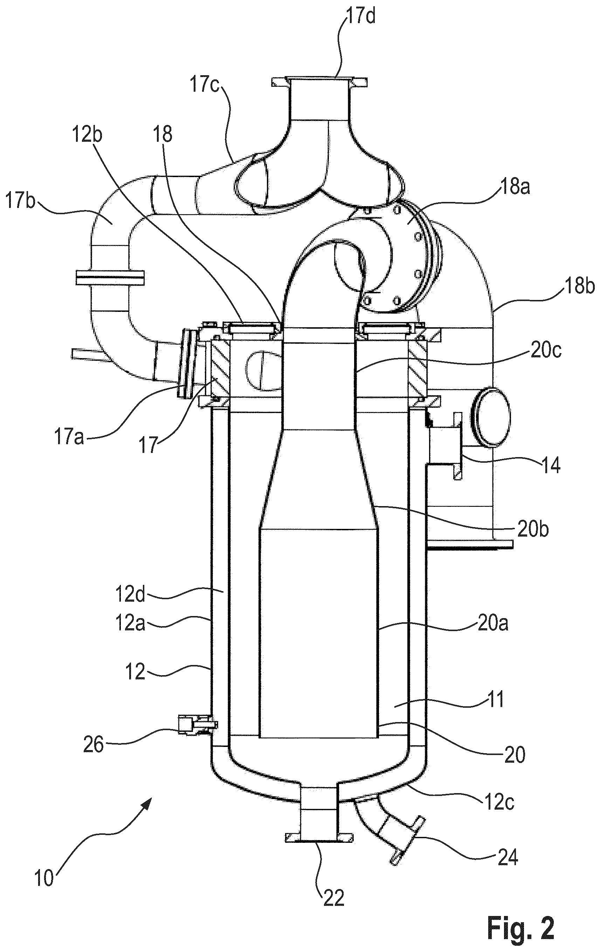

Fig. 2 stellt eine teilweise in vertikaler Richtung geschnittene Ansicht der in Fig. 1 dargestellten Vorrichtung 10 dar.

Im Unterschied zur perspektivischen Ansicht der Fig. 1, ist in Fig. 2 das Innere 11 des Gehäuses 12 zu sehen. Ein Hohlraum 11 wird von der Gehäusewand (dem Mantel) 12a und den beiden Stirnseiten 12b, 12c festgelegt. Die Gehäusewand 12a und die untere Stirnseite 12c sind doppelwandig ausgeführt mit einem dazwischenliegenden Zwischenraum 12d. Über den Heizanschluss 14 kann beispielsweise Wasserdampf in den Zwischenraum 12d eingebracht werden. Mit Hilfe des Wasserdampfes kann zumindest die Innenoberfläche des Gehäuses 12, d. h. zumindest die dem Hohlraum 11 zugewandte Oberfläche des Gehäuses 12 auf eine gewünschte Temperatur erwärmt bzw. aufgeheizt werden. Kondensat kann über den Auslass 24 aus dem Zwischenraum 12d abgelassen werden.

In Fig. 2 ist darüber hinaus ein Kanal (Auslasskanal) 20 zu sehen, der sich von der Auslassöffnung 18 in axialer Richtung des zylindrischen Gehäuses 12 in den Hohlraum 11 erstreckt. Der Auslasskanal 20 kann entlang seiner Längserstreckung, d. h. in axialer Richtung des Gehäuses 12, einen veränderlichen Durchmesser aufweisen. In Fig. 2 ändert sich der Durchmesser eines horizontalen Querschnittes entlang des Kanals. Von der Auslassöffnung 18 nimmt dieser Durchmesser entlang des Kanals 20 zu. Der Kanal 20 umfasst zu diesem Zweck zylindrisch ausgebildete Abschnitte 20a, 20c und zumindest einen kegelstumpfförmig ausgebildeten Abschnitt 20b. Gemäß Fig. 2 ist ein zylindrischer Kanalabschnitt 20c in die Auslassöffnung 18 dichtend eingesetzt. An den zylindrischen Kanalabschnitt 20c schließt sich außerhalb des Gehäuses 12 ein Rohrabschnitt mit endseitigem Flansch 18a an, der auch in Fig. 1 zu sehen ist. Innerhalb des Hohlraums 11 schließt sich ein kegelstumpfförmiger Abschnitt 20b an den zylindrischen Abschnitt 20c an, und ein weiterer zylindrischer Abschnitt 20a schließt sich an den kegelstumpfförmigen Abschnitt 20b an. Die Durchmesser der jeweiligen Abschnitte 20a, 20b, 20c sind so aufeinander abgestimmt, dass innerhalb des Auslasskanals 20 ein geschlossenwandiger Durchgang entsteht.

Die Länge bzw. Höhe H des Kanals 20, d. h. der Abstand H von der Auslassöffnung 18 bis zu dem in Fig. 2 gezeigten unteren Ende des Kanals 20, kann in einem Bereich von 0 < H < 0,9L liegen, wobei L die Länge des Hohlraumes 11 in vertikaler Richtung ist, die im Wesentlichen dem Abstand zwischen den beiden Stirnseiten 12b, 12c innerhalb des Hohlraumes 11 entspricht. Denkbar sind aber auch kürzere Längen H des Kanals, beispielsweise 0 < H < 0,8L, oder 0 < H < 0,7L, oder 0 < H < 0,6L, oder 0 < H < 0,5L. Es sollte innerhalb des Hohlraumes 11 ein ausreichend dimensionierter Freiraum zwischen dem Bereich zwischen der Innenwand des Gehäuses 12 und der Außenseite des Kanals 20 und dem Durchgang des Kanals 20 vorhanden sein.

Wird nun an diametral gegenüberliegenden Stellen der Gehäusewand 12a mit Druck beaufschlagte Luft durch die Öffnungen 16, 17 in den Hohlraum 11 des Gehäuses 12 eingebracht, entsteht ein sich mit hoher Geschwindigkeit rotierender Zyklon, der das ebenfalls in den Hohlraum 11 eingebrachte bzw. eingespritzte Wasserstoffperoxid in Kontakt mit der beheizten Innenoberfläche des Gehäuses 12 bringt, wo das Wasserstoffperoxid unmittelbar nach dem Auftreffen verdampft. Aufgrund der Geometrie des Auslasskanals 20 nimmt rein theoretisch die Drehgeschwindigkeit des Zyklons mit zunehmendem Abstand von den Einlassöffnungen 16, 17 in vertikaler Richtung nach unten zu, da der Abstand zwischen Innenoberfläche des Gehäuses 12 und der Außenseite des Kanals 20 innerhalb des Hohlraums 11 in vertikaler Richtung nach unten hin abnimmt und dadurch die sich schnell drehende Luft rein theoretisch weiter beschleunigt wird. Simulationen haben allerdings ergeben, dass die Drehgeschwindigkeit des Zyklon mehr oder weniger konstant gehalten werden kann, da etwaige Wandreibungseffekte an der Innenoberfläche des Gehäuses 12 und der Außenseite des Kanals 20 die sich rasch drehende Luft zu verlangsamen versuchen.

In Fig. 2 ist ebenso ein Temperaturfühler 26 zu sehen, dessen Fühlerspitze in den Zwischenraum 12d der doppelwandig ausgeführten Gehäusewand 12 ragt und auf diese Weise während des Betriebs der Vorrichtung 10 die Temperatur des Wasserdampfes innerhalb des Zwischenraumes 12d messen kann. Der Temperaturmesswert kann als Regelgröße einem externen Regelkreis zugeführt werden, der dafür sorgt, dass zumindest die Innenoberfläche des Gehäuses 12 auf einer erwünschten Zieltemperatur gehalten wird.

Um etwaige Ablagerungen innerhalb des Hohlraums 11 zu entfernen, die sich bei der Verdampfung von Wasserstoffperoxid aufgrund der darin enthaltenen Stabilisatoren an den Innenoberflächen des Gehäuses 12 und des Auslasskanals 20 bilden können, wird über den Reinigungsmittelanschluss 22 Reinigungsmittel in den Hohlraum 11 eingebracht, und zwar auf vorteilhafte Weise unter Druck, wodurch der Hohlraum 11 sich sowohl innerhalb des Auslasskanals 20 als auch in dem Zwischenraum zwischen dem Gehäuse 12 und der Außenseite des Auslasskanals 20 mit Reinigungsflüssigkeit füllt. Erreicht das Reinigungsmittel die Innenseite der oberen Stirnseite 12b des Gehäuses 12, und wird Reinigungsmittel weiterhin in den Hohlraum 11 eingebracht, gelangt das Reinigungsmittel über die Auslassöffnung 18 und die Rohrabschnitte 18a, 18b nach außen, wo es beispielsweise von einem Auffangbehälter aufgefangen werden kann. Denkbar ist auch, das Reinigungsmittel durch die Auslassöffnung 18 und die Rohrabschnitte 18a, 18b abzupumpen. Unabhängig davon, ob das Reinigungsmittel aus dem Hohlraum 11 abgepumpt wird oder nicht, kann auf diese Weise eine automatisierte Reinigung des gesamten Hohlraumes 11 und des Auslasskanals 20 sowie der sich an die Auslassöffnung 18 anschließenden Rohrabschnitte 18a, 18b bis zur nicht gezeigten Sterilisationskammer erzielt werden. Anstelle über die Auslassöffnung 18 kann der Hohlraum 11 auch über den Reinigungsmittelanschluss 22 automatisch bzw. automatisiert entleert werden. Der Reinigungsmittelanschluss 22 bietet zusätzlich noch die Möglichkeit, im Hohlraum 11 nicht verdampftes Wasserstoffperoxid abzulassen, wodurch ein etwaiges Gefahrenpotential durch angesammeltes flüssiges Wasserstoffperoxid vermieden werden kann.

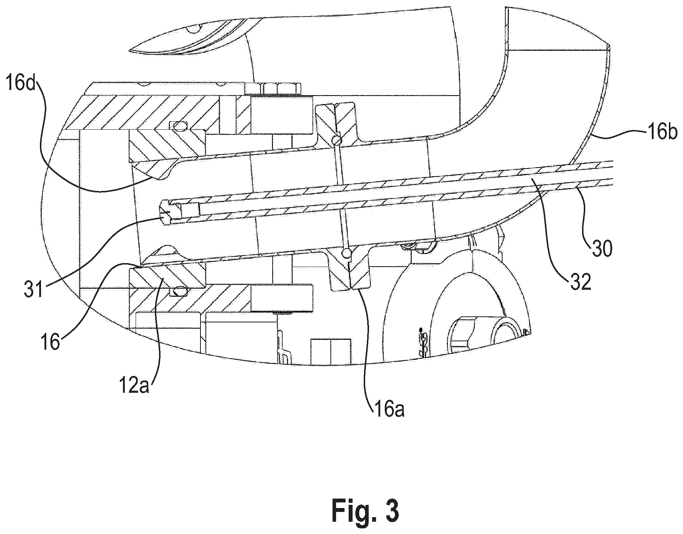

Fig. 3 stellt eine in vertikaler Richtung geschnittene Ansicht der in Fig. 1 dargestellten Vorrichtung 10 im Bereich der Einlassöffnung 16 für die Zuführung von mit Druck beaufschlagter Luft in das Innere 11 der Vorrichtung 10 dar.

Im Bereich der Einlassöffnung 16, an die sich der Rohrabschnitt 16a und daran ein weiterer Rohrabschnitt 16b anschließen, ist eine Düse 30, auf vorteilhafte Weise mittig, innerhalb des Rohrabschnittes 16a angeordnet. Die Düse 30 dient der Einbringung von flüssigem Wasserstoffperoxid in den Hohlraum 11 der Vorrichtung 10. Die Düse 30 ist zylindrisch ausgebildet und weist einen Durchgang 32 auf, der mit einem Reservoir, in dem Wasserstoffperoxid gespeichert ist, fluidmäßig verbunden ist. An dem der Einlassöffnung 16 zugewandten Ende der Düse 30 ist ein Düsenkopf 31 vorgesehen, durch den ein feiner Strahl Wasserstoffperoxid ausgegeben werden kann. Durch den Düsenkopf 31 wird das Wasserstoffperoxid bereits auf eine relativ hohe Geschwindigkeit beschleunigt, bevor es dann in den Hohlraum 11 der Vorrichtung 10 eintritt. Innerhalb des Rohrabschnittes 16a im Bereich des Düsenkopfes 31 ist eine Querschnittsverengung 16d vorgesehen, wodurch das Wasserstoffperoxid aufgrund der Querschnittsverengung (Venturi-Effekt) noch weiter beschleunigt wird.

Es versteht sich von selbst, dass eine entsprechende Düse 30 auf gleiche Weise auch in dem Rohrabschnitt 17a im Bereich der Einlassöffnung 17 angeordnet sein kann.

Wird nun mit Druck beaufschlagte Luft durch die Einlassöffnungen 16, 17 in den Hohlraum 11 des Gehäuses 12 eingebracht, bewirkt diese Luft eine Zerstäubung des Wasserstoffperoxidstrahls. Aufgrund der hohen Rotationsgeschwindigkeit der Luft (Zyklon) werden die Wasserstoffperoxidtröpfchen an die Innenoberfläche der Gehäusewand 12 gedrängt und dort verdampft. Die Druckverhältnisse innerhalb des Hohlraumes 11 des Gehäuses 12 und aufgrund der Tatsache, dass die Auslassöffnung 18 zur Atmosphäre hin offen ist, sorgen dafür, dass der Zyklon in der aufrechten Anordnung der Fig. 1 und der Fig. 2 nach unten gerichtet ist und folglich eine nahezu gleichmäßige Benetzung der Innenoberfläche des Gehäuses 12 stattfindet, wodurch die Verdampfung des Wasserstoffperoxids innerhalb des Hohlraumes 11 der Vorrichtung 10 weitestgehend gleichmäßig erfolgt und somit die Konzentration des verdampften Wasserstoffperoxids entlang der Zylinderachse des Gehäuses 12 sehr homogen ist. Erreicht der Zyklon den unteren Bereich des Gehäuses 12, wird dieser aufgrund der vorherrschenden Druckverhältnisse in den Auslasskanal 20 gedrängt, wo er sich bei der in Fig. 1 und Fig. 2 gezeigten aufrechten Anordnung der Vorrichtung 10 wieder nach oben bewegt und durch die Rohrabschnitte 18 A, 18 B schließlich eine Sterilisationskammer erreicht, die atmosphärischen Druck aufweist.

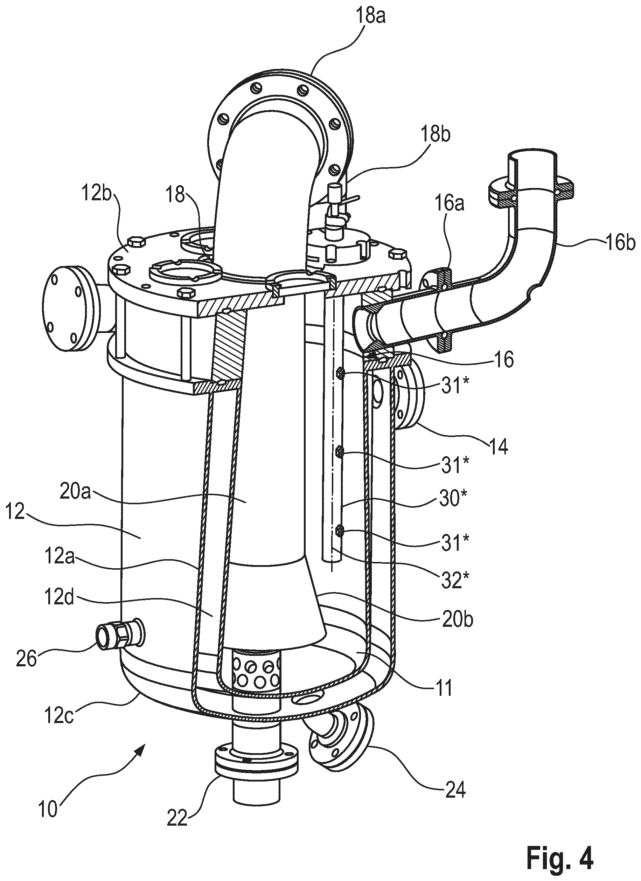

Fig. 4 stellt eine teilweise in vertikaler Richtung geschnittene Ansicht einer Vorrichtung gemäß einer noch weiteren bevorzugten Ausführungsform der Erfindung dar, die eine alternative Düse 30* zur Einbringung der zu verdampfenden Flüssigkeit in das Innere 11 der Vorrichtung 10 umfasst. Mit Ausnahme der Düse 30* sind die übrigen Komponenten der in Fig. 4 gezeigten Vorrichtung 10 identisch oder zumindest nahezu identisch zu den Komponenten der in Fig. 1 und Fig. 2 gezeigten Vorrichtung 10.

Gemäß Fig. 4 wird die zu verdampfende Flüssigkeit (z.B. Wasserstoffperoxid) nicht durch eine in der Gehäusewand 12a angeordneten Düse 30 (Fig. 3) in den Hohlraum 11 der Vorrichtung 10 eingebracht, sondern vielmehr über eine sich in vertikaler Richtung und entlang der Zylinderachse erstreckende Düsenlanze 30*, die eine Vielzahl von in vertikaler Richtung zueinander beabstandete Düsen 31* aufweist, welche in einem Abstand zur Gehäusewand 12a angeordnet ist. Die Vielzahl der Düsen 31* ist nicht auf drei beschränkt, wie dies in Fig. 4 dargestellt ist, sondern die Düsenlanze 30* kann auch mehr oder weniger als drei Düsen 31* umfassen. Im Innern der Düsenlanze 30* befindet sich eine in Fig. 4 lediglich schematisch angedeutete Versorgungsleitung 32*, die mit einem Wasserstoffperoxidreservoir fluidmäßig verbunden ist. Die Versorgungsleitung 32* versorgt sämtliche Düsen 31* mit Wasserstoffperoxid.

Die Anordnung der Düsen 31* innerhalb des Hohlraumes 11 ist so, dass sämtliche Düsen 31* in einem Abstand von der Innenoberfläche des Gehäuses 12 angeordnet sind, derart, dass ein aus den Düsen 31* austretender Wasserstoffperoxidstrahl senkrecht auf die Innenoberfläche der Gehäusewand 12 trifft. Auf diese Weise ist die Flugzeit der aus den Düsen 31* austretenden Wasserstoffperoxidstrahlen bis zum Auftreffen auf die beheizte Innenoberfläche der Gehäusewand 12 auf ein Minimum reduziert, wodurch eine Verdampfung des in den Hohlraum 11 eingespritzten Wasserstoffperoxidstrahls sehr rasch erfolgt. Wenngleich in Fig. 4 nicht zu sehen, kann zumindest eine der Düsen 31* auch als Venturi-Düse ausgebildet sein.

Denkbar ist ebenso, die Düsen 31* in einem Winkel zu den Druckluftstrahlen, die aus den Einlassöffnungen 16, 17 austreten, anzuordnen. Ebenso ist es denkbar, die Düsen 31* so anzuordnen, dass die aus ihnen austretende Behandlungsflüssigkeit entgegengesetzt zur Auslassrichtung der Druckluftstrahlen, die aus den Einlassöffnungen 16, 17 austreten, gerichtet ist.

Es versteht sich von selbst, dass der Einsatz einer Düsenlanze 30* die Verwendung einer wie in Fig. 3 dargestellten Düse 30 jeweils innerhalb der Rohrabschnitte 16a, 17a bei der in Fig. 4 gezeigten Vorrichtung 10 hinfällig macht.

Was den Auslasskanal in Fig. 4 betrifft, so ist dieser geringfügig anders gestaltet als der Auslasskanal 20 in der Fig. 2. In Fig. 4 ist der kegelstumpfförmige Abschnitt 20b an dem zum Hohlraum 11 hin offenen Ende des Auslasskanals 20 angeordnet, während in Fig. 2 ein zylindrischer Abschnitt 20a an dem zum Hohlraum 11 hin offenen Ende des Auslasskanals 20 angeordnet ist. In Fig. 4 ist aber auch ein entsprechend dem Auslasskanal 20 der Fig. 2 ausgebildeter Auslasskanal 20 denkbar, und umgekehrt auch.

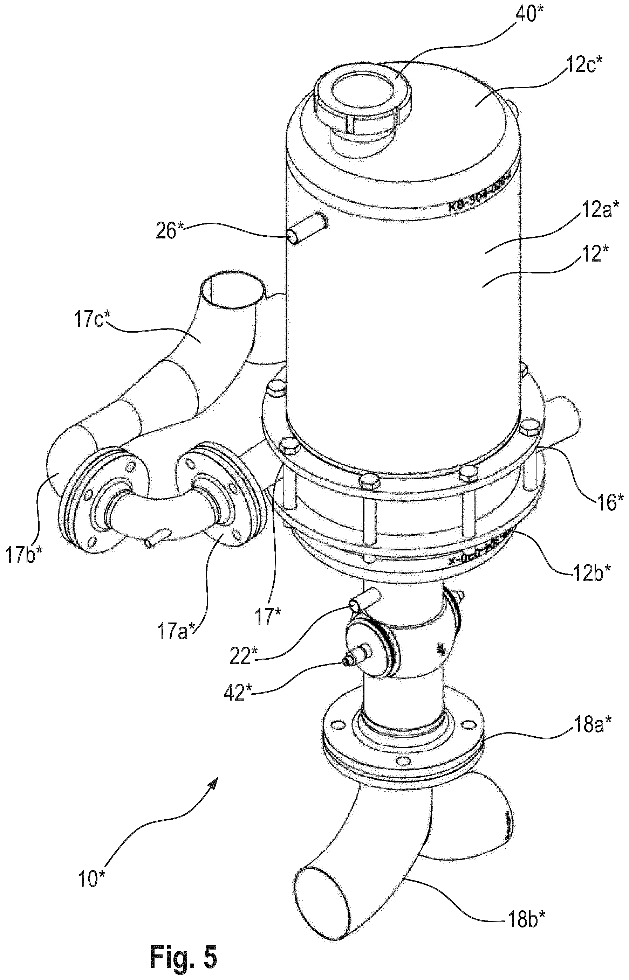

Fig. 5 ist eine schematische perspektivische Ansicht einer Vorrichtung zum Verdampfen einer Flüssigkeit, insbesondere einer Behandlungsflüssigkeit zur Sterilisation eines Behältnisses, gemäß einer weiteren bevorzugten Ausführungsform der Erfindung.

Fig. 5 stellt nun eine auf dem Kopf stehende Vorrichtung 10* dar, in der das verdampfte Wasserstoffperoxid durch die untere Stirnseite 12b* aus dem Inneren des Gehäuses 12* herausgeführt wird. Die Zuführung der mit Druck beaufschlagten Luft in den Hohlraum des Gehäuses 12* erfolgt dabei im Bereich der unteren Stirnseite 12b*, aber ebenso an diametral gegenüberliegenden Einlassöffnungen 16*, 17* und tangential zur Innenoberfläche des Gehäuses 12*. Ein Schauglas 40* befindet sich an der oberen Stirnseite 12c*. Ein Temperaturfühler 26* ist ebenso vorgesehen.

Bei der in Fig. 5 gezeigten Vorrichtung 10* kann entweder die in Fig. 3 gezeigte Düse 30 oder die in Fig. 4 dargestellte Düsenlanze 30* zur Einspritzung von Wasserstoffperoxid in den Hohlraum des Gehäuses 12* verwendet werden.

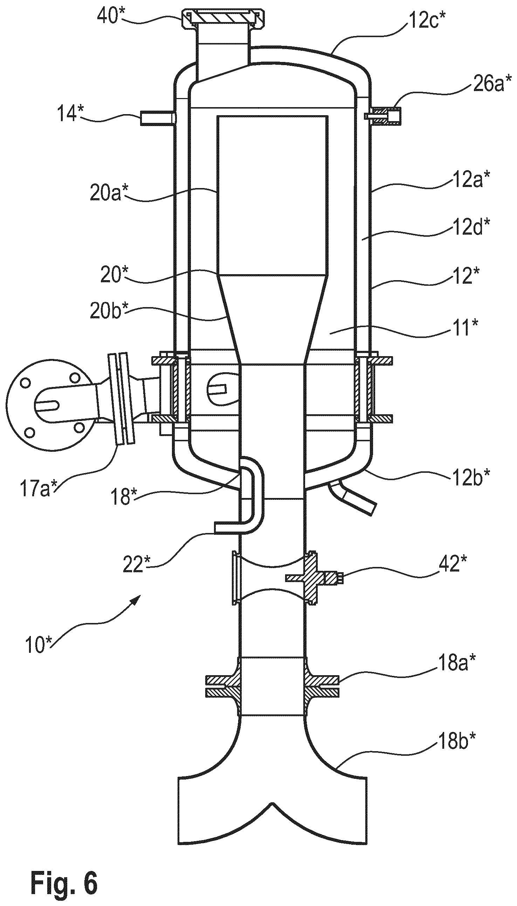

Fig. 6 zeigt eine in vertikaler Richtung geschnittene Ansicht der in Fig. 5 dargestellten Vorrichtung 10*. Da die in Fig. 6 gezeigte Vorrichtung 10* umgekehrt aufrecht angeordnet ist, sind die Einlassöffnungen 16*, 17* im unteren Bereich des Gehäuses 12* und im Bereich der unteren Stirnseite 12b* angeordnet.

Im Unterschied zu der in den Fig. 1, 2 und 4 gezeigten Vorrichtung 10, bei denen die obere Stirnseite 12b nicht beheizt wird, wird bei der Vorrichtung 10* das gesamte Gehäuse 12*, d.h. die Gehäusewand 12a*, die obere Stirnseite 12c* und die untere Stirnseite 12b* mittels Dampf beheizt. Auch bei dieser Ausführungsform ist das Gehäuse 12* doppelwandig ausgeführt, jedoch mit einem durchgehenden (umlaufenden) Zwischenraum 12d*, wodurch zumindest die dem Hohlraum 11* zugewandte Innenoberfläche des Gehäuses 12* vollständig auf eine gewünschte Temperatur aufgeheizt werden kann. Durch den Heizanschluss 14* wird Dampf in den Zwischenraum 12d* eingebracht.

In vertikaler Richtung nach unten gesehen, umfasst der Auslasskanal 20* einen oberen zylindrischen Abschnitt 20a*, daran anschließend einen kegelstumpfförmigen Abschnitt 20b* sowie einen weiteren zylindrischen Abschnitt 20c*, der einen kleineren Durchmesser besitzt als der zylindrische Abschnitt 20a*. Der zylindrische Abschnitt 20c* ist dichtend in die Auslassöffnung 18* eingesetzt. Die Durchmesser sämtlicher Abschnitte 20a*, 20b* und 20c* sind aufeinander derart abgestimmt, dass sie einen geschlossenen Durchgang festlegen.

Der Reinigungsmittelanschluss 22* bietet die gleiche oder zumindest funktionsgleiche Funktion wie der Reinigungsmittelanschluss 22 der in den Fig. 1 und 2 gezeigten Vorrichtung 10. Um etwaige Ablagerungen innerhalb des Hohlraums 11* zu entfernen, die sich bei der Verdampfung von Wasserstoffperoxid aufgrund der darin enthaltenen Stabilisatoren an den Innenoberflächen des Gehäuses 12* und des Auslasskanals 20* bilden können, wird über den Reinigungsmittelanschluss 22* Reinigungsmittel in den Hohlraum 11* eingebracht, und zwar auf vorteilhafte Weise unter Druck, wodurch sich zunächst der Zwischenraum zwischen dem Gehäuse 12* und der Außenseite des Auslasskanals 20* mit Reinigungsmittelflüssigkeit füllt. Erreicht das Reinigungsmittel die obere Kante des Auslasskanals 20*, gelangt das Reinigungsmittel in den Auslasskanal 20* und über die Auslassöffnung 18* in die Rohrabschnitte 18a*, 18b*, von wo es dann nach außen, beispielsweise in einen Auffangbehälter, fließt. Denkbar ist auch, das Reinigungsmittel durch die Auslassöffnung 18* und die Rohrabschnitte 18a*, 18b* abzupumpen. Unabhängig davon, ob das Reinigungsmittel aus dem Hohlraum 11* abgepumpt wird oder nicht, kann auf diese Weise eine automatisierte Reinigung des gesamten Hohlraumes 11* und des Auslasskanals 20* sowie der sich an die Auslassöffnung 18* anschließenden Rohrabschnitte 18a*, 18b* bis zur nicht gezeigten Sterilisationskammer erzielt werden. Anstelle über die Auslassöffnung 18* kann der Hohlraum 11* auch über den Reinigungsmittelanschluss 22* automatisiert entleert werden.

Der Reinigungsmittelanschluss 22* bietet zusätzlich noch die Möglichkeit, im Hohlraum 11* nicht verdampftes Wasserstoffperoxid aus dem Hohlraum 11* abzulassen, wodurch ein etwaiges Gefahrenpotential durch angesammeltes flüssiges Wasserstoffperoxid vermieden werden kann.

Mit Druck beaufschlagte Luft wird durch die Einlassöffnungen 16*, 17* in den Hohlraum 11* des Gehäuses 12* eingebracht und bewirkt eine Zerstäubung der aus den Düsen 30 oder 30* austretenden Wasserstoffperoxidstrahlen. Aufgrund der hohen Rotationsgeschwindigkeit der Luft (Zyklon) werden die Wasserstoffperoxidtröpfchen an die Innenoberfläche der Gehäusewand 12* gedrängt und dort verdampft. Die Druckverhältnisse innerhalb des Hohlraumes des Gehäuses 12* und aufgrund der Tatsache, dass die Auslassöffnung 18* (Fig. 6) zur Atmosphäre hin offen ist, sorgen dafür, dass der Zyklon in der umgekehrt aufrechten bzw. auf dem Kopf stehenden Anordnung der Fig. 6 nach oben gerichtet ist und folglich eine nahezu gleichmäßige Benetzung der Innenoberfläche des Gehäuses 12* stattfindet, wodurch die Verdampfung des Wasserstoffperoxids innerhalb des Hohlraumes 11* der Vorrichtung 10* weitestgehend gleichmäßig erfolgt und somit die Konzentration des verdampften Wasserstoffperoxids entlang der Zylinderachse des Gehäuses 12* sehr homogen ist. Erreicht der Zyklon den oberen Bereich des Gehäuses 12*, wird dieser aufgrund der vorherrschenden Druckverhältnisse in den Auslasskanal 20* gedrängt.

Innerhalb des Auslasskanals 20* bewegt sich die Luft samt dem verdampften Wasserstoffperoxid nach unten und durch die Rohrabschnitte 18a*, 18b* und erreicht sodann eine Sterilisationskammer, die atmosphärischen Druck aufweist.

Am Rohrabschnitt 18a* ist ein in das Innere des Rohrabschnittes 18a* hineinragender Temperaturfühler 42* vorgesehen, der die Temperatur des Gemisches aus Druckluft und verdampften Wasserstoffperoxid, das durch den Auslasskanal 20* und durch die Auslassöffnung 18* strömt, misst. Die Temperatur ist ein Indiaktor dafür, ob die Temperatur innerhalb des Hohlraumes 11*, und damit die Temperatur an der Innenoberfläche des Gehäuses 12* ausreicht, um das eingespritzte Wasserstoffperoxid möglichst vollständig zu verdampfen. Anstelle eines Temperaturfühlers 42* ist ebenso die Verwendung eines Detektors möglich, der verdampftes Wasserstoffperoxid (in ppm) erfassen kann.

## Patentansprüche

1. Vorrichtung (10) zum Verdampfen einer Flüssigkeit, insbesondere einer Behandlungsflüssigkeit zur Sterilisation eines Behältnisses, umfassend:
ein Gehäuse (12), das einen Hohlraum (11) festlegt;
einen Heizanschluss (14), mit Hilfe dessen zumindest eine dem Hohlraum (11) zugewandte Innenoberfläche des Gehäuses (12) zumindest abschnittsweise beheizbar ist;
mindestens eine Einlassöffnung (16, 17) zum Zuführen von mit Druck beaufschlagter Luft in den Hohlraum (11);
mindestens eine Düse (30) zum Zuführen einer Behandlungsflüssigkeit in den Hohlraum (11), wobei bei bestimmungsgemäßer Verwendung der Vorrichtung (10) die Behandlungsflüssigkeit bei Kontakt mit der beheizten Innenoberfläche des Gehäuses (12) verdampft;
eine Auslassöffnung (18) zum Abführen der verdampften Behandlungsflüssigkeit aus dem Hohlraum (11); und
einen Kanal (20), der einen Durchgang festlegt und sich von der Auslassöffnung (18) in den Hohlraum (11) erstreckt,
**dadurch gekennzeichnet, dass** der Kanal (20) sich bei bestimmungsgemäßer Verwendung der Vorrichtung (10) in vertikaler Richtung erstreckt.

2. Vorrichtung (10) nach Anspruch 1, wobei der Kanal (20) in einem Querschnitt senkrecht zur Längserstreckung des Kanals (20) eine Umfangslänge besitzt, die entlang des Kanals (20) variiert.

3. Vorrichtung (10) nach Anspruch 2, wobei die Umfangslänge entlang des Kanals (20) mit zunehmendem Abstand von der Auslassöffnung (18) zunimmt.

4. Vorrichtung (10) nach einem der vorhergehenden Ansprüche, wobei der Kanal (20) abschnittsweise zylinderförmig (20a) und/oder abschnittsweise kegelstumpfförmig (20b) ist.

5. Vorrichtung (10) nach einem der vorhergehenden Ansprüche, wobei der Kanal (20) sich über eine Längserstreckung L_{Kanal} erstreckt, die in einem Bereich von 0 < L_{Kanal} < 0,9L_{Hohlraum}, wobei der Wert L_{Kanal} die Länge des Kanals gemessen von der Auslassöffnung (18) und L_{Hohlraum} die Länge der Längserstreckung des Hohlraumes (11) ist.

6. Vorrichtung (10) nach einem der vorhergehenden Ansprüche, des Weiteren umfassend eine Reinigungsmitteleinlassöffnung (22) zum Zuführen von Reinigungsmittel in den Hohlraum (11).

7. Vorrichtung (10) nach Anspruch 6, wobei der Durchgang des Kanals (20) der Reinigungsmitteleinlassöffnung (22) zugewandt ist.

8. Vorrichtung (10) nach einem der vorhergehenden Ansprüche, wobei die mindestens eine Einlassöffnung (16, 17) mit einem Einlassrohr (16a, 16b) verbunden ist, das mit einer Einrichtung zur Bereitstellung von mit Druck beaufschlagter Luft fluidmäßig verbunden ist, und wobei die mindestens eine Düse (30) koaxial und innerhalb des Einlassrohres (16a, 17a) angeordnet ist.

9. Vorrichtung (10) nach Anspruch 8, wobei das Einlassrohr (16a, 17a) oder die mindestens eine Einlassöffnung (16, 17) im Bereich der mindestens einen Düse (30) eine Querschnittsverengung (16d) aufweist.

10. Vorrichtung (10) nach einem der vorhergehenden Ansprüche, wobei die mindestens eine Einlassöffnung (16, 17) so angeordnet ist, dass die mit Druck beaufschlagte Luft, welche die mindestens eine Einlassöffnung (16, 17) verlässt, tangential zur Innenoberfläche des Gehäuses (12) in den Hohlraum (11) eintritt.

11. Vorrichtung (10) nach einem der vorhergehenden Ansprüche, wobei mindestens zwei gegenüberliegende Einlassöffnungen (16, 17) vorgesehen sind, und wobei jede Einlassöffnung (16, 17) der mindestens zwei Einlassöffnungen (16, 17) so angeordnet ist, dass die mit Druck beaufschlagte Luft, welche die Einlassöffnung (16, 17) verlässt, tangential zur Innenoberfläche des Gehäuses (12) in den Hohlraum (11) eintritt.

12. Vorrichtung (10) nach einem der Ansprüche 1 bis 7, wobei die mindestens eine Düse (31*) in einem Abstand von der Innenoberfläche des Gehäuses (12) innerhalb des Hohlraumes (11) so angeordnet ist, dass ein die mindestens eine Düse (30*) verlassender hypothetischer Düsenstrahl senkrecht auf die Innenoberfläche des Gehäuses (12) trifft.

13. Vorrichtung (10) nach Anspruch 12, wobei eine Vielzahl von Düsen (31*) an einer Düsenlanze (30*) angeordnet ist, und wobei die Düsenlanze (30*) sich bei bestimmungsgemäßer Verwendung der Vorrichtung (10) in vertikaler Richtung erstreckt und innerhalb des Hohlraumes (11) angeordnet ist, und wobei die Vielzahl der Düsen (31*) in einem Abstand von der Innenoberfläche des Gehäuses (12) angeordnet ist.

14. Vorrichtung (10) nach Anspruch 13, wobei die Vielzahl von Düsen (31*) an der Düsenlanze (30*) so angeordnet ist, dass eine Vielzahl von hypothetischen Düsenstrahlen, die aus der Vielzahl von Düsen (31*) austreten, senkrecht auf die Innenoberfläche des Gehäuses (12) treffen.

15. Vorrichtung nach Anspruch 13 oder 14, wobei sämtliche Düsen (31*) der Düsenlanze (30) mit lediglich einer innerhalb der Düsenlanze (30*) verlaufenden Versorgungsleitung (32*) fluidmäßig verbunden sind.

16. Vorrichtung (10) nach einem der Ansprüche 13 bis 15, wobei die Düsenlanze (30*) eine Kühlleitung aufweist zum Kühlen der Düsenlanze (30*).

17. Vorrichtung (10) nach einem der vorhergehenden Ansprüche, wobei das Gehäuse (12) zumindest abschnittsweise doppelwandig mit einem Zwischenraum (12d) ausgebildet ist, und wobei der Heizanschluss (14) mit dem Zwischenraum (12d) fluidmäßig verbunden ist zum Einführen von Wasserdampf in den Zwischenraum (12d) und zum zumindest abschnittsweise Aufheizen von zumindestens der Innenoberfläche des Gehäuses (12).

18. Vorrichtung (10) nach Anspruch 17, des Weiteren umfassend einen Temperaturfühler (26), dessen Fühlerspitze sich in den Zwischenraum (12d) des doppelwandigen Gehäuses (12a) erstreckt.

19. Vorrichtung (10) nach einem der vorhergehenden Ansprüche, wobei das Gehäuse (12) zylindrisch ausgebildet ist.

20. Vorrichtung (10) nach einem der vorhergehenden Ansprüche, wobei die mindestens eine Düse (30) zum Zuführen von Wasserstoffperoxid in den Hohlraum (11) ausgebildet ist.
